# EUROPEAN PATENT SPECIFICATION

(11) **EP 1 827 229 B1**
(45) Date of publication and mention of the grant of the patent: **24.08.2011**
(21) Application number: 05815062.4
(22) Date of filing: 28.10.2005
(51) Int. Cl.: A61B 5/103, G03B 29/00

(54) **APPARATUS FOR AND METHOD OF TAKING AND VIEWING IMAGES OF THE SKIN**
GERÄT UND VERFAHREN ZUR AUFNAHME UND BETRACHTUNG VON HAUTBILDERN
APPAREIL ET PROCEDE PERMETTANT DE SAISIR ET DE VISIONNER DES IMAGES DE LA PEAU

(30) Priority: 29.10.2004 US 978284
(43) Date of publication of application: 05.09.2007
(73) Proprietor: Johnson and Johnson Consumer Companies, Inc., Skillman, NJ 08558 (US)
(72) Inventor: MEROLA, Kenneth, Agoura Hills, CA 91301 (US); KOLLIAS, Nikiforos, Skillman, NJ 08558 (US); POTE, Kenneth, Easton, PA 18042 (US); PAYONK, Gregory, Flanders, NJ 07836 (US)
(74) Representative: Kirsch, Susan Edith
(86) International application number: PCT/US2005/038951
(87) International publication number: WO 2006/050104

(56) References cited:
- EP-A- 1 433 418
- US-A- 4 911 544
- US-A1- 2004 146 290

## Description

### CROSS-REFERENCES TO RELATED APPLICATIONS

This is a continuation in part of Application Serial No. 10/008,753, entitled Method Of Taking Images Of The Skin Using Blue Light And The Use Thereof; filed November 8, 2001 and owned by the assignee hereof.

### FIELD OF THE INVENTION

The present invention relates to an apparatus for and method of taking and viewing images of the skin, and more particularly for taking multiple images using a plurality of different wavelengths of light, viewing and using the images.

### BACKGROUND OF THE INVENTION

In order to promote skin care products, many cosmetic companies ask their potential customers questions regarding perception of their skin. Based on the answers to these questions, cosmetic companies are able to better suggest cosmetic and therapeutic products to these people. Examples of such promotions can be found on the Internet WebPages of Neutrogena® (www.neutrogena.com), L'Oreal® (www.lorealparisusa.com), and Lancome® (www.lancome.com). These questions, however, are only based upon the subject's perception of their skin under visible light. Many skin problems, however, are not always visible under such conditions.

Various types of photography have been developed to enhance the visualization of the skin. In visible light photography, or standard photography, the most common arrangement includes a camera and one or more flash units to deliver visible light to the skin by direct illumination, diffuse illumination, or a combination thereof. Angled lighting has also been used to generate a gradient of the illuminating field on the skin in order to enhance the visualization of wrinkles and fine lines. Depending on the direction of the gradient (vertical or horizontal), different sets of wrinkles and fine lines may be visually enhanced.

Polarized light photography has also been developed to selectively enhance either surface or subsurface features of the skin. These results are accomplished by placing a polarizing filter (typically a linear polarizing filter) both in front of the flash unit, and in front of the camera. When the polarizing filters are in the same orientation with each other, surface features of the skin such as scales, wrinkles, fine lines, pores, and hairs are visually enhanced. When the polarizing filters are aligned perpendicular to each other, subsurface features of the skin such as erythema, pigmentation, blood vessels, and hair, are visually enhanced.

Ultraviolet photography, where the flash unit is filtered to produce ultraviolet A light and the camera is filtered so that only visible light enters the lens, has been used to visually enhance the appearance of pigmentation, the bacteria *p. acnes*, and horns. A variation of ultraviolet photography has been termed the "sun camera" where ultraviolet A light is used to illuminate the skin and an ultraviolet A sensitive film or a digital camera is used to record the reflected ultraviolet light from the skin. In this arrangement, both the pigment distribution and the surface features of the skin are visually enhanced.

The present invention provides people with access to one or more of these improved means of viewing their skin, in order to provide them additional insight into the condition of their skin. Such insight allows them to make more informed decisions regarding the purchase of skin care products. In addition, skin care products can be suggested to such potential customers by retailers or professionals based upon such customer's enhanced perception of their skin.

Patent application US 2004/146290 A1 discloses an apparatus according to the preamble of claim1.

### SUMMARY OF THE INVENTION

The problems and disadvantages associated with conventional apparatus and techniques utilized to capture and review images are overcome by the present invention, which includes an apparatus and method for capturing images of a person as claimed. The apparatus has a housing, a cameras mounted in the housing for capturing the images of the person a and means for illuminating mounted in the housing for providing light for capturing the images of the person. The illumination means is capable of providing at least two different wavelengths of light in capturing the images. The wavelengths of light used in capturing a first images differ from the wavelengths of light used in capturing a subsequent image. The apparatus includes a display mounted in the housing for displaying images captured by the camera; and a controlled for controlling the camera, the illumination means and the display for selectively capturing and displaying the captured images non the display.

In accordance with a method of the invention., an imaging station having a housing, a camera, a light source, a display mounted in the housing for displaying images captured by the camera and a computer is provided. The person whose image is to be captured is positioned relative to the camera and a plurality of images is automatically captured with the camera. The captured images are stored and selectively displayed on the display under operator control.

Other aspects, features, and advantages of the present invention will be apparent from the detailed description of the invention and from the claims.

### BRIEF DESCRIPTION OF THE DRAWINGS

FIG. 1 is an overhead view of an apparatus used to sequentially take the following four types of pictures of a person: a standard photograph, a polarized photograph, an ultraviolet A photograph, and a blue fluorescence photograph.
FIG. 2 is a perspective view of an alternative embodiment of the present invention.
FIG. 3 is a diagrammatic top view of the present invention shown in FIG. 2, in particular, showing an angular orientation of a pair of doors thereof relative to a central housing thereof.
FIG. 4 is an elevational view of a touch screen display of the present invention shown in FIG. 2.
FIGS. 5 through 8 are flow charts showing exemplary processing flows associated with the operation and control of the present invention shown in FIGS. 2 through 4.
FIG. 9 is an exploded view of the present invention as shown in FIGS. 2 through 4.
FIG. 10 is a perspective view of the present invention shown in FIGS 2 through 4 and 9 with the doors closed.
FIG. 11 is a perspective view of the present invention shown in FIG. 10, viewed from the rear.
FIG. 12 is a block diagram showing the system components of the present invention shown in FIGS. 2 through 11.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference.

### The Camera

Various types of cameras may be used in the methods of the present invention. Examples of such cameras include, but are not limited to, standard 35mm cameras, cameras using instant developing film (such as those available from Polaroid Corporation, Cambridge, Massachusetts USA), and digital cameras. Preferably, a digital camera is used as it provides fast access to the images taken of the subject. It also allows the image to be displayed on a large monitor, enables the subject to easily enlarge areas of skin that are of particular interest (e.g., areas of the face), and allows the image to be printed in a report which can also include suggestions for products addressing any concerns the subject noticed upon examining the images. Examples of suitable digital cameras include, but are not limited to, those which take images of at least 1 million pixels, preferable at least 4 million pixels. Examples of such digital cameras include, but are not limited to, the Nikon D1X (Nikon, Tokyo, Japan) and the Fuji S1 (Fuji, Tokyo, Japan).

One or more cameras may also be used in the methods of the present invention, e.g., separate cameras having a distinct light filtering lens may be used for each type of photograph taken and/or separate cameras used to photograph different areas or angles of the subject. Preferably, only one camera is used since having more than one camera would require that the cameras be calibrated to have the same color and intensity response. When only one camera is used, a mechanical filter wheel or arm containing a filter(s) may be placed in front of the camera to selectively filter the light prior to or after entering the camera's lens and/or the respective filter(s) may be placed at the light source(s) to filter the light as it leaves the light source(s). In the case where multiple light sources are used, the camera can communicate with each of the respective light sources via hard wiring or a radio transceiver.

In one embodiment, the camera(s) are mounted at the same level as the area of skin that the subject desires to be photographed, e.g., the face of the subject. Preferably, the camera is set such that such area of skin substantially fills the frame area of the photograph, e.g., to ensure the greatest amount of detail in the image.

In one embodiment where multiple images are acquired by single camera, the images are preferably acquired in less than about 30 seconds, e.g., less than about 10 seconds.

### Standard Photography

In one embodiment, the method includes the step of taking a standard photograph of the subject. What is meant by "standard photograph" a photograph that is taken of the subject using visible light (e.g., light having a wavelength from about 400 to about 700 nm). In one embodiment, the subject is illuminated with one or more, preferably two, flash units that emit visible light. In one embodiment, the flash unit(s) are further equipped with a diffusing filter that is placed in front of each flash unit. A diffusing filter is a filter, which assists in uniformly dispersing light (e.g., to help eliminate "hot spots"). Examples of such diffusing filters include, but are not limited to, frosted glass filters such as a Broncolor Diffuser (Sinar Bron, Allschwil, Switzerland), metal grids which may be printed on glass substrates, or a diffusing reflective umbrella for indirect lighting.

In one embodiment, the flash unit(s) are angled at the subject's skin to generate a gradient across the surface of the skin. In a further embodiment, the flash units are mounted higher than the skin area of the subject and aimed at such skin area in order to give a gradient of light on the skin from the top to the bottom. In one embodiment, the angle of the flash units is from about 5 to about 30 degrees, such as about 10 degrees, from horizontal. This gradient visually enhances various features of the skin such as the fine lines and wrinkles in the subject, e.g., the crow's feet around the eye and forehead or mouth area wrinkles.

### Polarized Light Photography

In one embodiment, the method includes the step of taking a polarized photograph of the subject. What is meant by "polarized photograph" is a photograph of the subject taken (i) with a light source that emits light through a polarizing filter and/or (ii) through a polarized filter that filters light prior to or after entering the camera's lens.

In one embodiment, the camera and one or more flash units, preferably two, are on about the same plane as the subject's skin to be photographed, and the flash units are placed so that the angle formed by each flash unit(s), subject's skin, and camera is about 35 to 55 degrees, such as about 45 degrees. In one embodiment, a polarizing filter is placed in front of each flash unit. What is meant by a "polarizing filter" is a filter that filters incoming light to emit substantially only polarized light. What is meant by the term "substantially," as used herein, is at least 75 percent, preferably 90 percent, and most preferably at least 95 percent.

Examples of a polarizing filter include, but are not limited to, polarizing plates such as those available from Edmund Scientific (Barrington, NJ USA), polarizing prisms such as Glan Thomson polarizing prisms, or a polarizing reflector that reflects light at about the Brewster angle. Polarizing filters may be linear or circular polarizing filters. In a further embodiment, a light diffuser is placed between the flash unit and the polarizing filter.

In one embodiment, a linear polarizing filter is used at the light source and the linear polarizing filter is arranged such that the electric field of the emitted light is about perpendicular to the plane formed by the light source, the person's skin, and the camera. In another embodiment, a linear polarizing filter is used at the light source and the linear polarizing filter is arranged such that the electric field of the emitted light is about parallel to the plane formed by the light source, the person's skin, and the camera.

In a further embodiment, the flash unit(s) are positioned on a horizontal plane with the camera and the subject's skin and the polarizing filter is a linear polarizing filter oriented so that the electric field of the transmitted light is in the vertical direction (e.g., perpendicular to the plane). In this orientation, the critical angle for total internal reflection from within the top corneocytes is 45 degrees, thereby resulting in an image that is dominated by the light thus reflected from the corneocytes. The resulting image has a high degree of glare, which is further enhanced when an optical coupling medium, such as sebum or "oils," is present on the surface of the corneocytes. The polarized image, thereby, allows an estimate to be made as to the oiliness of the subject's skin. It also provides insight into the number and severity of pores on the cheek and forehead areas of the facial skin. Other desired outcomes of polarized photography include, but are not limited to, an enhanced image of surface features such as fine lines, skin texture, scales and vellous hair.

In another embodiment, the flash unit(s) are positioned on a vertical plane above the camera and the subject's skin so that the angle formed by the flash units, subject's skin, and camera is about 35 to 55 degrees such as about 45 degrees and flash unit(s) are filtered with linear polarizing filter that is placed with the transmitted electric field in the vertical direction (e.g., parallel to the plane). In this arrangement the surface glare from the skin is minimized, thus, enhancing the subsurface features of the skin, such as erythema (redness), blood vessels, and pigmentation.

Polarized light sources on both on the horizontal and vertical planes with the camera and the subject's skin can be used to enhance specific aspects of the skin (e.g., the face) that are partially shaded with the use of polarized light sources only on the horizontal or vertical planes alone.

In one embodiment, the photograph of the subject is taken both with a light source that emits lights through a polarizing filter and through a polarizing filter that filters the light prior to or after entering the camera's lens. When the polarizing filters are in the same orientation with each other (e.g., both horizontal or both vertical), surface features of the skin such as scales, wrinkles, fine lines, pores, and hairs are visually enhanced. When the polarizing filters are aligned perpendicular to each other (e.g., one horizontal and one vertical), subsurface features of the skin such as erythema, pigmentation, blood vessels, and hair, are visually enhanced.

### Ultraviolet Photography

In one embodiment, the method includes the step of taking an ultraviolet photograph of the subject. What is meant by "ultraviolet photograph" is a photograph of the subject taken (i) with a light source that either emits substantially only ultraviolet light (radiation) or emits light through an ultraviolet filter and/or (ii) through an ultraviolet filter that filters the light prior to or after entering the camera's lens. What is meant by an ultraviolet filter is a filter that filters incoming light to emit substantially only ultraviolet light (e.g., light having a wavelength from about 200 to about 400 nm). Examples of light sources that can emit substantially only ultraviolet light are light emitting diodes. Examples of ultraviolet photography include, but are not limited to, ultraviolet A photography or ultraviolet B photography.

### Ultraviolet A Photography

In one embodiment, the method includes the step of taking an ultraviolet A photograph of the subject. What is meant by "ultraviolet A photograph" is a photograph of the subject taken (i) with a light source that emits substantially only ultraviolet A light or emits light through an ultraviolet A filter and/or (ii) through an ultraviolet A filter that filters the light prior to or after entering the camera's lens.

In one embodiment, one or more, preferably two, flash units are filtered with an ultraviolet A filter ("UVA filter"). What is meant by a UVA filter is a filter that filters incoming light to emit substantially only light having a wavelength of from about 320 to about 400 nm. Examples of UVA filters include, but are not limited to, the ultraviolet UG-11 filter (Schott Glass Technologies, Duryea, Pennsylvania USA). The resulting image may be rich in red color because of the long wavelength pass of UVA filter. In one embodiment, when utilizing a digital camera, either the blue or green channel, preferably the blue channel, of the RGB image is selected for viewing, resulting in a black and white image.

Benefits of an ultraviolet A photograph include, but are not limited to, enhanced appearance of pigmented macules on the skin and surface features such as bumps and wrinkles. Ultraviolet A photography may be used to determine the uniformity of application of topical products, such as sunscreens and of make-ups, that contain materials that absorb ultraviolet radiation. In addition, since melanin pigmentation more strongly absorbs UVA radiation than visible light, illuminating the skin with UVA radiation gives an enhanced contrast between normal skin and hyper pigmented skin. Furthermore, the pigmented macules are visualized as dark spots on a bright background due to the scattering and the fluorescence of the dermal collagen matrix. The image recorded by the camera includes both the reflection of ultraviolet A radiation and fluorescence of the collagen. The resulting black and white image obtained by the blue or green channel from a digital camera provides an enhanced view of the distribution of pigmented macules on the skin (e.g., the face). For subjects with deeply pigmented skin, the red channel may be selected.

In another embodiment, the flash units are further filtered with a red blocking filter. Examples of such red blocking filter include, but is not limited to, a KG-5 filter (Schott Glass Technologies). Such filters may assist in correcting the red appearance of the image.

### Blue Fluorescence Photography

In one embodiment, the method includes the step of taking a blue fluorescence photograph of the subject. What is meant by "blue fluorescence photograph" is a photograph of the subject taken with a light source that emits substantially only blue light or emits light through a blue filter. What is meant by "blue light" is light having a wavelength from about 380 to about 430 nm.

In one embodiment, one or more, preferably two, flash units are filtered with a blue filter. What is meant by a "blue filter" is a filter that filters incoming light to emit substantially only blue light. Examples of such blue filters include, but are not limited to, interference filters such as those available from Melles Griot (Irvine, CA USA) or dielectric filters.

In one embodiment, the light entering the camera is also filtered (e.g., prior to or after entering the lens of the camera) with a long pass filter to substantially eliminate light having a wavelength below about 400 nm. Examples of long pass filters include, but are not limited to, GG-420 or GG-440 filters (Schott Glass Technologies) and Kodak Wratten No. 8 (Eastman Kodak, Rochester, NY USA). In one embodiment, the flash units and filters are placed on either side of the camera at approximately the same horizontal plane as the skin sample of the subject.

This type of photography produces bright images of the distribution of coproporphyrin produced by the bacteria *P. acnes* and of horns. What is meant by a "horn" is a mixture of sebaceous lipids, keratinocytes, and possibly sebocytes impacted in open comedones and blackheads on the skin. By using substantially only blue light that is within the Soret absorption band of porphyrins, the fluorescence emission of coproporphyrin is maximized. Excitation in this range also yields bright emission images of the distribution of "horns" because the fluorescence yield of horns is higher when excited in the blue region of the spectrum.

In one embodiment, when utilizing a digital camera, the color image may be viewed showing the distribution of coproporphyrin and therefore the sites of maximum *p. acnes* concentration, which appears red in the image. The image also contains bright white spots, which correspond to clogged pores or open comedones. In another embodiment the green channel of the RGB image is selected to enhance the horn fluorescence emission and the red channel may be selected to enhance the fluorescence emission of porphyrins from *p. acnes*. The resulting black and white images, thus, provide excellent imaging of small vessels because hemoglobin has its Soret band in the same wavelength range as porphyrins. In one embodiment, these vessels are visualized using either the blue or the green channel of the RBG image.

### Promotion of Skin Care Products

Upon acquisition of the photographs, these images are presented to the subject. The means of presenting the photographs depends in part on the type of photograph taken (e.g., using standard film, instant developing film, or a digital image). When using standard film or instant developing film, the prints of the images are provided to the subject. The prints may also be scanned and presented to the subject on a computer monitor (e.g., a LCD or CRT monitor). When using a digital camera, the image may also be presented on such a monitor.

Following presentation of the images to the subject, skin care products can be suggested to the person based upon his/her review of the images. In one embodiment, the method comprises presenting the subject with one or more questions relating to the presented images. Based upon the answers to such questions, one or more skin care products can be suggested to the subject. These products can be associated with responses to the questionnaire, made by a person reviewing the subject's answers, or made by a computer based upon the answers of the subject. The review of the various images by the subject facilitates more informed answers to the questions.

In one embodiment, the suggestions of skin care products is made by a computer program that recommends products based upon the answers provided by the subject. In one embodiment, a list of skin care products are maintained on a relational database. These products are associated with answers to certain questions. Thus, based upon the answers provided by the subject, certain products are selected by the computer program. For example, if the subject answers that he/she has wrinkles, the computer program will search the data based for skin care products effective for treating wrinkles (e.g., products containing retinol) and/or if the subject answers that he/she has acne, the computer program will search the database for skin care products effective for treating acne (e.g., products containing benzoyl peroxide or salicylic acid).

In one embodiment, these suggestions are limited to a set number of products, e.g., the program will not recommend more than five products. In such a case, the computer program will prioritize skin care product suggestions based upon either the importance of the skin disorders identified by the subject or the database's ranking of importance of the skin disorder to be addressed. For example, if the subject responds that he/she has severe acne and moderate fine lines, the computer program will recommend acne product(s).

In one embodiment, following application of a skin care product (e.g., one suggested by the present method) for a period of time (e.g., one week, one month, or one year), the subject is then photographed again. These new photographs are compared to the original photographs to determine the efficacy of the skin care product.

In one embodiment, the recommended products may be available at the location where the photographs are taken, e.g., the photographs are taken in a store or kiosk that sells skin care products.

### Skin Care Product

Following the subject's visual analysis of the images, skin care product(s) can be suggested to the subject to address any perceived problems identified following such analysis.

What is meant by a "skin care product" is a topical composition comprising cosmetically active agent. What is meant by a "cosmetically active agent" is a compound (e.g., a synthetic compound or a compound isolated from a natural source) that has a cosmetic or therapeutic effect on the skin, including, but not limiting to, anti-aging agents, lightening agents, darkening agents such as self-tanning agents, anti-acne agents, shine control agents, anti-microbial agents, anti-inflammatory agents, antimycotic agents, anti-parasite agents, sunscreens such as UVA/UVB blocking or absorbing agents, photo protectors, antioxidants, keratolytic agents, detergents/surfactants, astringents, moisturizers, nutrients, amino acids, amino acid derivatives, minerals, plant extracts, animal-derived substances, vitamins, energy enhancers, anti-perspiration agents, astringents, deodorants, hair removers, hair growth stimulators, hair growth retarding agents, firming agents, anti-callous agents, and agents for nail and/or skin conditioning.

In one embodiment, the cosmetically-active agent is selected from, but not limited to, the group consisting of hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, retinoids such as retinoic acid, retinol, and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, lipoic acid, amino acids such a proline and tyrosine, vitamins, lactobionic acid, acetylcoenzyme A, niacin, riboflavin, thiamin, ribose, electron transporters such as NADH and FADH2, and other botanical extracts such as aloe vera, witch hazel, and legumes such as soy beans, and derivatives and mixtures thereof. The cosmetically active agent will typically be present in the composition of the invention in an amount of from about 0.001% to about 20% by weight of the composition, e.g., about 0.01% to about 10% such as about 0.1% to about 5%.

Examples of vitamins include, but are not limited to, vitamin A, vitamin Bs such as vitamin B3, vitamin B5, and vitamin B12, vitamin C, vitamin K, and vitamin E and derivatives thereof.

Examples of hydroxy acids include, but are not limited, to glycolic acid, lactic acid, malic acid, salicylic acid, citric acid, and tartaric acid.

Examples of antioxidants include but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, glutathione, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

Various other cosmetically-active agents may also be present in the skin care products. These include, but are not limited to, skin protectants, humectants, and emollients. The skin care products may also comprise chelating agents (e.g., EDTA), preservatives (e.g., parabens), pigments, dyes, opacifiers (e.g., titanium dioxide), and fragrances.

The following is an example of a manner of practicing a method of the present invention. Other manners may be practiced by those of ordinary skill in the art.

### Example

One embodiment of the present invention utilizes a kiosk that is intended to be an interactive tool from which subjects (e.g., potential customers) can evaluate their facial skin and decide upon a course of action to improve the appearance of the skin. The kiosk is designed such that a subject will have a series of images acquired of their face and the images will be presented to them one at a time along with questions reflating to the displayed images.

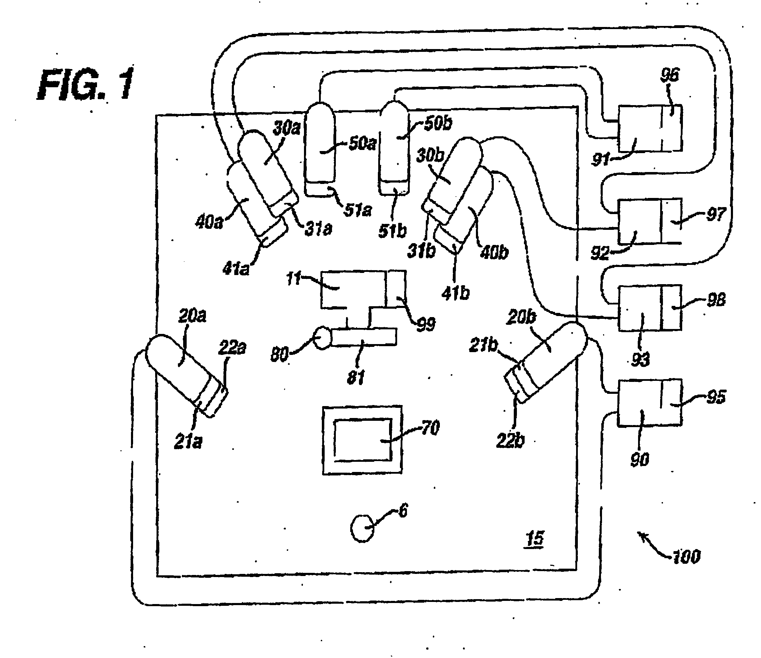

In one example of the present invention, the kiosk comprises apparatus 100, as set forth in Fig. 1 (not to scale). Apparatus 100 is enclosed above and on three side (the side containing chin rest 6 in open for the subject to enter his/her head) with a frosted plastic glass (not shown). Apparatus 100, which is capable of taking four types of photographs of the subject, is set-up on table 15 having dimensions of 30" by 36". Half way along the long dimension of table 15 (about 18" from one end) and about 1 5/8" in from the front end of table 15 there is chin rest 6 for the subject's chin. The height of chin rest 6 is about 12" above table 15. Across from chin rest 6 and exactly half way along the opposite end of the table 15 is camera 11 (Nikon D1X). Camera 11 is mounted so that the center of the camera lens of camera 11 is about 17" above the top of table 15. The distance between chin rest 6 and the front end of the lens of camera 11 is adjusted so that the subject's face substantially fills the camera frame of camera 11.

On the side of table 15 away from chin rest 6 are flash units 30a, 30b, 40a, 40b, 50a, and 50b (Broncolor Picolites, Sinar Bron, Allschwil, Switzerland) which are powered, respectively, by power packs 92, 92, 93, 93, 91, and 91. The standard flash units 50a and 50b, which are used for taking a standard photograph, are mounted above camera 10 and angled down about 20 degrees. Flash units 50a and 50b are directed toward the center of the subject's face. Diffusing filters 51a and 51b (Broncolor Diffuser, Sinar Bron) are placed, respectively, in front of flash units 50a and 50b.

UVA flash units 30a and 30b, which are used for the ultraviolet A photography, are mounted on either side of camera 11 at about 14" from the edge of table 15 and at a height of about 20" from the top of table 15. UVA filters 31a and 31b (UG-11 filters, Schott Glass Technologies, Duryea, Pennsylvania USA) are placed, respectively, in front of UVA flash units 30a and 30b. Blue flash units 40a and 40b, which are used for blue fluorescence photography, are also mounted on either side of the camera at about 14" from the edge of the table top and at a height of about 13" from the top of table 15. Blue filters 41a and 41b (Melles Griot, Irvine, California USA) are placed, respectively, in front of blue flash units 40a and 40b. The UVA flash units 30a and 30b and the blue flash units 40a and 40b are directed to the center of the face of the subject.

The polarized flash units 20a and 20b (Broncolor Picolites), which are used for polarized light photography, are powered by power pack 90. Diffusing filters 21a and 21b (Broncolor Diffuser, Sinar Bron) are placed, respectively, in front of polarized flash units 20a and 20b, respectively. Linear polarizing filters 22a and 22b (Edmund Scientific, Barrington, New Jersey USA) are placed, respectively, in front of diffusing filters 21a and 21b in a vertical orientation. Polarized flash unit 20a is positioned at about 4 1/2" from the left edge and about 14" in from the proximal edge of table 15 and polarized flash unit 20b is positioned at about 4 1/2" from the right edge and about 14" in from the proximal edge of table 15. The angle between either flash units 20a or 20b, the chin rest 6, and the camera 11 is about 45 degrees.

The method begins when the subject enters the kiosk image acquisition area and enters basic demographic information into a facial skin-care evaluation computer program (Microsoft Visual Basic, Microsoft Corporation, Redmond, WA USA) using a touch-screen monitor 70 (SecurePoint, SeePoint Technologies, Torrance, CA), which is mounted under table 15 and connected to the same computer running the computer program. The subject enters data into the computer program via monitor 70 (computer program runs MountFocus Runtime Keyboard and the keyboard present on monitor 70 was designed using MountFocus Keyboard designer programs (MountFocus Information Systems, Wilmington, DE USA)), however, other input device such as a keyboard, a track ball, and a computer mouse may be used. Examples of such information include, but are not limited to, age and gender of the subject. Following the input of such demographic information, the computer program then instructs the subject to place their chin on chin rest 6 and indicates to the subject to close his/her eyes since apparatus 100 is ready to take photographs.

Upon touching monitor 70, the software makes a function call to an image acquisition and display software ("IDL software"; IDL, Research Systems, Inc., Boulder, CO) running on the same computer. The IDL software then triggers camera 11 to acquire a blue fluorescence photograph, a standard photograph, a polarized photograph, and an ultraviolet A photograph. The flash units 40a, 40b, 50a, 50b, 20a, 20b, 30a, and 30b are triggered sequentially through the use of a radio transceiver (Pocket Wizard Multimax, LPA Design, South Burlington, VT USA) using transceivers 95 (attached to power pack 90 and operating in receiver mode), 96 (attached to power pack 91 and operating in receiver mode), 97 (attached to power pack 92 and operating in receiver mode), 98 (attached to power pacK 99 and operating in receiver mode), and 99 (attached to the hot shoe of camera 11 and operating in transmitter mode). A Topas A2 power pack (Sinar Bron) is used for power packs 90 and 91 and a Primo 4 power pack (Sinar Bron) is used for power packs 92 and 93. The radio transceiver causes the activation of the pairs of flash units in response to the shutter release of camera 11.

Prior to taking the blue fluorescence photograph, the IDL software makes a call to servo motor 80, using a Mini SSC II circuit board (Scott Edwards Electronics, Sierra Vista, AZ USA), to move long pass filter 81 (Kodak Wratten No. 8, Eastman Kodak, Rochester, NY USA) in front of the lens of camera 11. After this movement, the blue fluorescence photograph is taken. Following the taking of this photograph, the IDL software then makes another call to servo motor 80 to move long pass filter 81 away from the lens of camera 11. The IDL software then instructs the camera to take the remaining three images. Apparatus 100 is able to acquire these four images in about 10 seconds.

At this point, the four images just acquired are stored in the memory of camera 11 as separate data files. The IDL software then makes function calls to these data files and requests these files be transferred to the computer running the computer software and saved to its hard disk with a file name that indicates the apparatus used, subject identifier, and the type of image.

The subject is then presented on monitor 70 with a registration form. Examples of such questions include e-mail address, places where they buy skin-care products, ethnic background, and amount and type of skin-care products that they have purchased in the past (e.g., the past year).

The subject then begins reviewing each of the four images on monitor 70 and answers questions, presented on monitor 70, about each image. The computer program calls the IDL software and requests that a particular saved image be loaded from the hard disk and resized to fit in the screen display area. Once the image is displayed, the IDL software then returns control to the computer program. The facial image display area is roughly half of monitor 70. The other half of the screen of monitor 70 displays a series of questions relating to the particular displayed image. To assist the subject in the review of his/her images, the computer program may also display on monitor 70 images of other people as comparisons.

As the subject advances to the next page, the computer program calls IDL program and requests that a particular saved image be loaded from the hard disk and resized to fit in the screen of monitor 70 area as discussed above. This procedure continues until all four images have been displayed and all questions have been answered by the subject.

Upon viewing the standard photograph, the subject is then presented with questions regarding the surface of his/her skin. Examples of such questions include, but are not limited to, whether they have any fine lines, wrinkles, loss of elasticity or firmness, large visible pores, sensitive skin, and rough or smooth skin, as well as the severity and location of such conditions. Other questions include, but are not limited to, the frequency and severity of irritation from skin care products.

Upon viewing the polarized photograph, the subject is asked questions regarding the oiliness of their skin. Examples of such questions include, but are not limited to, whether they have normal, dry, oily, or selective oily skin (e.g., oil in certain areas such as above the eyebrows and on the tip of the nose).

Upon viewing the ultraviolet A photograph, the subject is asked questions regarding the visualization of pigmentation of the face (e.g., brown spots). Examples of such questions include, but are not limited to, the amount and location of such pigmented spots.

Upon viewing the blue fluorescence photograph, the subject is asked questions regarding acne. Examples of such questions include, but are not limited to, the severity and frequency of his/her breakouts.

As described above, the images from camera 11 are displayed on the computer monitor 70. However, because the number of available screen pixels are less than the number of actual image pixels taken by the digital photograph, only a small percentage of the original image can actually be displayed if the image is to be shown on the screen in its entirety while maintaining aspect ratio. For the case of the Nikon D1X, which stores 6 million pixels per image, display of the digital image in a portrait orientation on a computer screen having a resolution of 1024 X 768 results in display of only 1 out of every 18 pixels. In such a down-sampled image presentation, fine detail of the skin taken by camera 11 is not fully displayed.

The solution to this problem is to use a technique whereby a 256 x 256 box (display kernel) appears directly over the area of interest and shows all the image pixels actually acquired by camera 11 for such area. The effect is an in-place magnification of a small area of the image on monitor 70. The apparent magnification, shown as this display kernel, can be passed over various areas of the image selected by the subject. Thus, this is an example of 1:1 image display (where every image pixel is shown on the screen within a small display kernel). True magnification of the image can also be accomplished by interpolating the data between pixels and creating additional pixels, thereby providing magnification of select areas of the image. The subject Selects the magnification of such area by touching the area of interest displayed on the monitor 70.

Following the input of the answers from the subject, the computer program then proceeds to suggest skin care products for the subject. Each question in the computer program is associated with a skin condition. For example, the question "How often does your skin breakout?" is associated with acne. As the subject answers each question, the corresponding skin condition is assigned a degree. For example, the acne question has four degrees corresponding to the four answer choices: Always, Weekly, Monthly and Never.
After the subject answers all the questions, the skin conditions questioned by the computer program are ranked according to severity. This ranking is accomplished by passing all of the degree values entered by the subject to a relational database stored procedure contained within a relational database (Microsoft SQL, Microsoft Corporation, Redmond, WA USA) that is on the same computer.

A database table contained with the relational database contains a record for each skin problem type and degree. A "degree weight" is assigned to each record, which facilitates the ranking of the skin conditions. For example, if the subject answers "Always" for the above acne question, and "Yes" to the question "Do you notice any loss of firmness on your face?" the acne skin condition may have a higher rank than the loss of firmness condition. However, if the subject answers "Weekly" to the above acne question and "Yes" to the loss of firmness question, the loss of firmness condition may be ranked higher.

After the skin conditions are ranked, the top three conditions, along with the subject's skin type, e.g., normal, dry, or oily, are passed to another relational database stored procedure contained within the relational database. Using these values, this procedure queries a second database table that contains all of the possible combinations of skin conditions along with skin type. Each such record in the table contains a list of recommended products based on these values. This corresponding list of recommended products for that subject's condition is then passed back to the computer program.

Finally, the computer program creates a printout using Crystal Reports (Seagate Corporation, Scotts Valley, CA USA) for the subject including the suggested skin care products and pictures of the subject with information about the various skin conditions. At the conclusion of computer program, the subject will indicate whether or not the images should be kept or deleted.

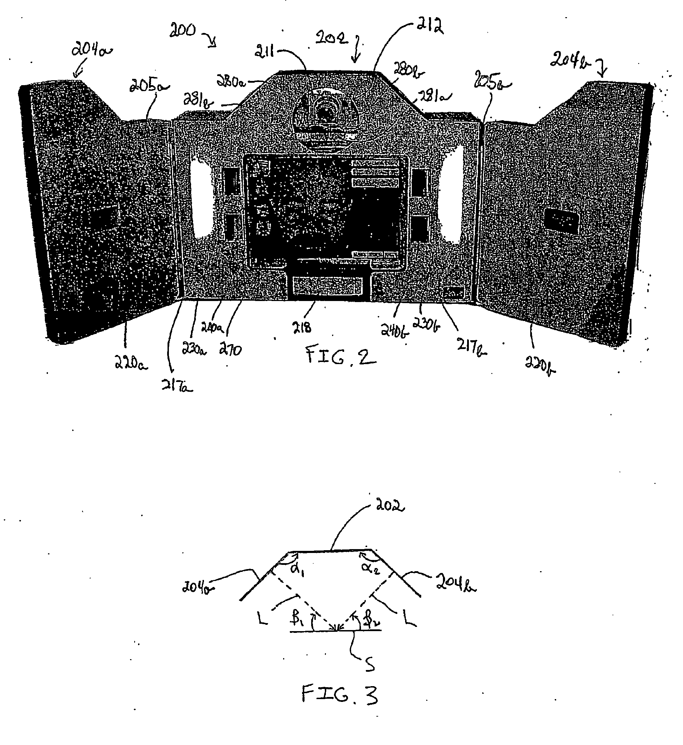

FIGs. 2 and 3 show an alternative embodiment of the present invention wherein imaging station 200 is provided in the form of a cabinet unit with a central housing 202 flanked by a pair of hingedly connected door assemblies 204a, 204b. The door assemblies 204a, 204b can be opened and closed relative to the central housing 202. FIG. 3 shows that the angle between the door assemblies 204a, 204b and the central housing 202 can be adjusted to adjust the angle of incident light cast on subjects by flash units mounted to the door assemblies 204a, 204b.

The angular orientation α1, α2 of door assemblies 204a, 204b relative to the central housing 202 results in light L1, L2 emanating from flash units 220a, 220b, respectively, having an angle of incidence of β1 and β2, relative to the subject S. As noted above, the incidence angle of the light illuminating a subject S, can be selected to aid in capturing images which are more probative of skin condition, e.g., when capturing polarized light images.

The angles α1, α2 can be controlled by conventional means, such as an adjustable, threaded stop or a slotted, pivoting bracket coacting with a limit pin, as are commonly known for controlling the extent of opening of hingedly mounted doors and lids. In this manner an optimal angle for one or both door assemblies 204a, 204b is repeatable.

The embodiment of the present invention shown in FIG. 2 shares many features of the previously described kiosk embodiment and includes a camera 211 (preferably digital) placed within a camera niche 212 for capturing the images of a subject S. As noted above with respect to the kiosk embodiment, a number of different illuminating lights (flashes) are available for capturing images. More particularly, the imaging station 200 has fluorescent lights 217a, 217b, polarized flashes 220a, 220b, a pair of UVA flashes 230a, 230b having a wavelength of, e.g., 405 nanometers. A pair of UV flashes 240a, 240b having a wavelength, e.g., of 385 nanometers are positioned below the 405 nanometer flashes 230a, 230b. The flashes 230a, 230b and 220a, 220b may optionally be operated in conjunction with filters 281a, 281b that can be rotated in front of the camera lens by solenoids 280a, 280b to further select the wavelengths of light received by the camera 211. For example, a yellow filter 281a may be rotated in front of the camera 211 by solenoid 280b when taking pictures with the 405 nanometer flashes 230a, 230b. A polarizing filter 281b may be used in conjunction with polarized flashes 220a, 220b.

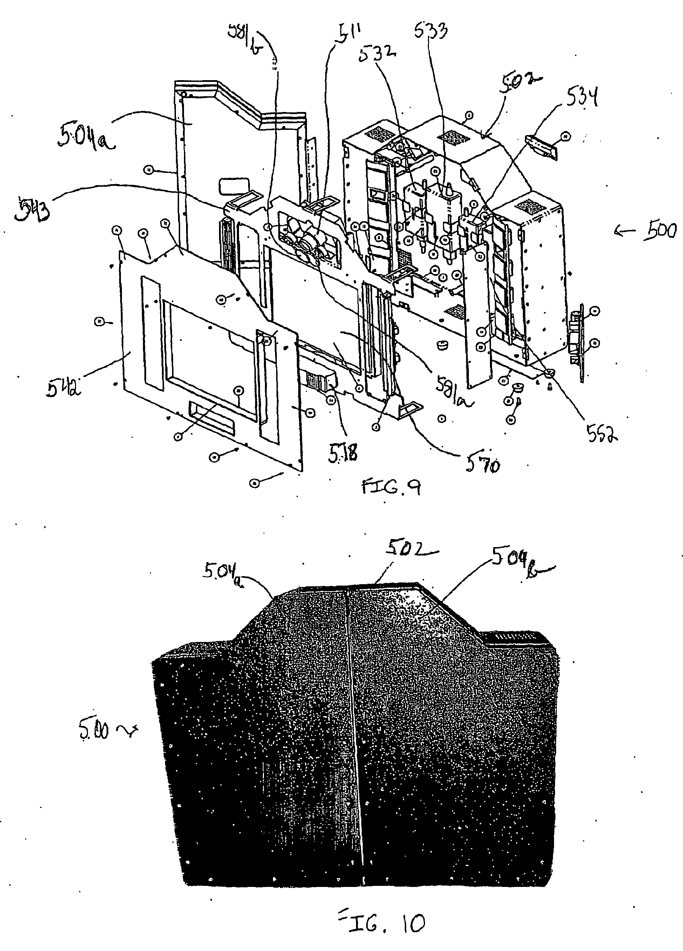

Various combinations of illuminating light(s) and filter(s) may be used for image capture. The present invention is not limited to the number and placement of flashes and filters shown. More particularly, a greater or lesser number of filters and flashes may be employed, e.g., see FIG. 9. While rotating filters 281a, 281b are described above, non-moving filters can be placed over one or more flashes to control the light wavelengths that are used to capture an image. Multiple standard flashes may be employed in conjunction with a variety of filters to achieve illumination under a variety of selected wavelengths of light. Combinations of flashes may be activated simultaneously to acquire a single image or sequentially to acquire multiple, sequential images. It should be noted that the use of multiple flashes with different filter options may be preferable under certain circumstances than utilizing a moveable filter. That is, by simplifying the imagining station 200 and reducing the number of moving parts, the imagining station 200, may be made more cheaply and function more reliably.

A touch screen monitor 270 is disposed below the camera 211 and displays the photographic images recently obtained by the camera 211 and/or previously obtained images that reside in memory or on a storage device, such as a computer disk or memory stick. A speaker 218 is provided in the imaging station 200 for announcing instructions to the user (subject S). The speaker 218 is particularly useful in giving instructions to the subjects when their eyes are closed, e.g., immediately prior to, during and immediately after a photographic session, when flashes are activated to capture images of the subject's face.

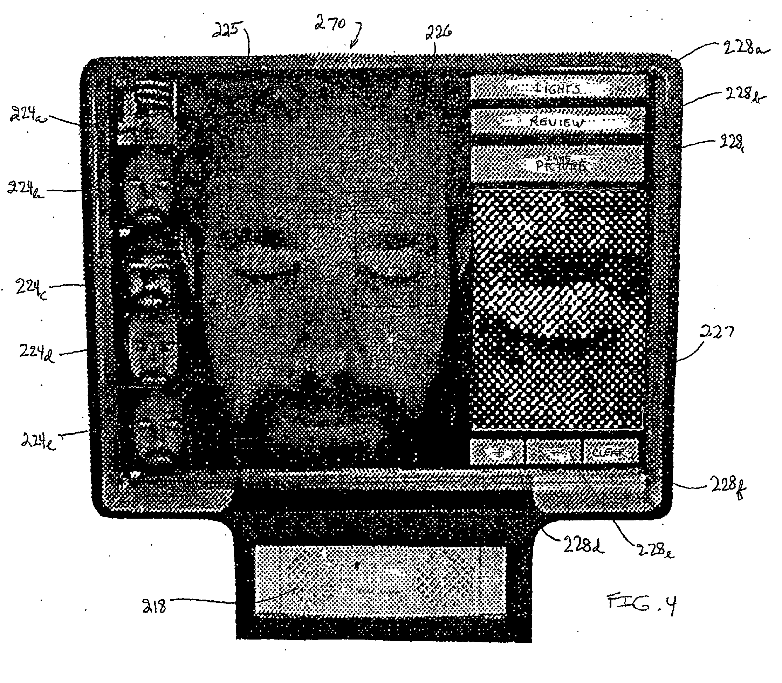

FIG. 4 shows an enlarged view of the monitor 270 which is preferably a touch screen monitor allowing the simultaneous display of information and the processing of control inputs from the operator/user/subject S who touches the screen to select control options. In this manner, the operator may control the images displayed to review and study them for the purposes of diagnosing and evaluating skin condition. At the left side of the display monitor 270 a plurality of thumbnail images 224a through 224e may be displayed. The first thumbnail image 224a may be utilized to display (both in thumbnail and in enlarged format) a real time moving image captured by the camera 211 in video mode which permits the user to interactively view and position their face in front of the camera for centering and focusing and optimally capturing their facial image. The real time image is therefore, in some respects, like a mirror. To initiate an imaging session, a computer program may be used to inform the user, either through visually displayed instructions or auditory instructions, to position their face such that it substantially fills the display frame and is centered therein.

The remaining thumbnail images 224b through 224e may be used to display images that have been captured during an imaging session utilizing different wavelengths of illuminating light. As described above with respect to the kiosk embodiment, the imaging station 200 preferably takes a sequence of images utilizing different wavelengths of light, via different flashes and filters, etc. in order to capture images which reveal different attributes of the subject's skin. Selecting one of the thumbnail images 224b through 224e by touching it will cause that particular image to be displayed in the main display area 225 on a larger scale Optionally, the thumbnail images 224a-224e may be labeled with text indicating the type of image that it is, e.g., "Blue Light" or "White Light" and/or the time and date when taken. Selecting a thumbnail image of a particular type may invoke instructions and observations from the computer in the imaging station 200 which relate to that particular type of image. For example, the computer program may announce a particular type of image has been selected and provide tips on what is shown by this type of image - "You have selected a blue light image. This type of image is particularly useful in assessing condition(s) X, Y, Z. In making this assessment, look for A, B, C in the image, which are indicative of D, E, F..." This type of message can be delivered in text on the display and/or in audio form over speaker 218. The delivery of this type of message is preferably enabled/disabled under operator control, e.g., by touching a control button, "Enable/Disable Instructional Messaging" or an equivalent control means. The foregoing highlights that in order to effectively employ the imaging station 200, the observer of the captured image is preferably educated in the technique of observing and interpreting features apparent in the captured images. The pertinent information for effectively using the imaging station 200 may be communicated to the user/subject S by the above-noted messaging, but also by written and/or recorded materials provided with the station 200 (e.g. in CD ROM or video form). In the event that the imaging station is connected to the Internet, information can be provided to the user/subject S interactively by retrieving the information from a website and presenting it to the user/subject S at appropriate times or by presenting hyper links that the user/subject may activate based upon interest. By surveying the main image area 225, the user can ascertain the presence or absence of certain skin conditions revealed by the particular light that was used in capturing the image. The thumbnail images therefore provide an intuitive and transparent means for identifying and reviewing a plurality of images taken during an imaging session. While the imaging method described above suggests that multiple images are captured by multiple sequential exposures of a static subject S with different wavelengths of light, the present invention may also be utilized to take one or more pictures after the subject S had varied their position. For example, a series of polarized images may be captured with the subject being instructed to rotate their face from left silhouette, to a forward facing position, to a right silhouette position, or in varying degrees of looking up, down and straight ahead. While the present invention is very useful for examining the face, images of other surfaces of the body may also be captured, recorded and analyzed. For example, the status and progress of a burn, abrasion, rash, melanoma, mole or other skin condition on any part of the body may be observed and analyzed using the present invention.

On the upper right hand portion of the display monitor 270, a plurality of virtual control buttons 228a-228c are displayed and in the lower right hand portion, control buttons 228d-228f are displayed. Control button 228a controls the fluorescent lighting 217a, 217b (on/off and/or dim down/brighten), which illuminates the subject S in a darkened room and allows the subject S to position his or her face before the camera (as shown in thumbnail 224a). (In order to control the wavelengths of light in which images are taken, it is preferable to have reduced ambient illumination, e.g., due to room lighting). Control button 228b is utilized to review old images taken at a prior photo session. By reviewing images taken at different times, a user can compare the progress of their skin condition over time. Preferably, the imaging station 200 permits the selection of a plurality of images to be displayed, e.g., two or three, in large scale, juxtaposed next to one another. This permits side-by-side comparison to observe trends in skin condition over time or to compare images taken under different lighting conditions, e.g., comparing a polarized light image to a white light image. Control button 228c may be used to initiate a photo session after the user has positioned themselves properly before the camera 211. A prompt may be provided instructing the user/subject S to press control button 228c after they have positioned themselves.

An enlarged (magnified kernel) display area 227 displays a portion of the main image area 225 at a greater level of magnification. The portion that is magnified is determined by focus box 226, which is overlaid on the image displayed in the main display area 225. The purpose of the enlarged display area 227 is to focus upon a specific area of the image shown in the main display area 225 for closer examination, at a greater magnification. Accordingly, the user may position the focus box 226 over any area of the image shown in display area 225 by touching the monitor/screen 270 in the area delimited by the focus box 226 and "dragging" the focus box 226 to the area of interest on the image in the main display area 225. The corresponding portion of the image bounded by the focus box 226 is then displayed in the enlarged display area 227. The image portion shown in enlarged display area 227 may then be further enlarged or reduced (zoom in and out) by the control buttons 228d and 228e ("+" indicating zoom in for greater magnification and "-" indicating zoom out for lesser magnification). The enlarged display area 227 may also be cleared by touching control button 228f.

As noted above, the comparison of side-by-side images may at times be instructive. This comparative process using, e.g., a pair of side-by-side images of a person's face taken under the same lighting conditions but at different times or at the same time but using different lighting, can be further enhanced by performing zooming and focusing functions on one or both images. One embodiment involves coordinated or synchronized focusing and zooming. More particularly, when viewing a pair of images, the focus box graphic appearing in each of the images may be located on the same place in each image (e.g., on the tip of the nose). When the focus box is moved on one of the images, an equal displacement of the focus box on the other image is effected. For example, if the focus box on both of the images is on the tip of the nose and the user/subject S moves the focus box on one of the images to the eye area, then the focus box on the other image moves to the eye area also. Zooming can also be coordinated in the same manner, such that when comparing side-by-side images, the focus area and magnification are the same, promoting comparison.

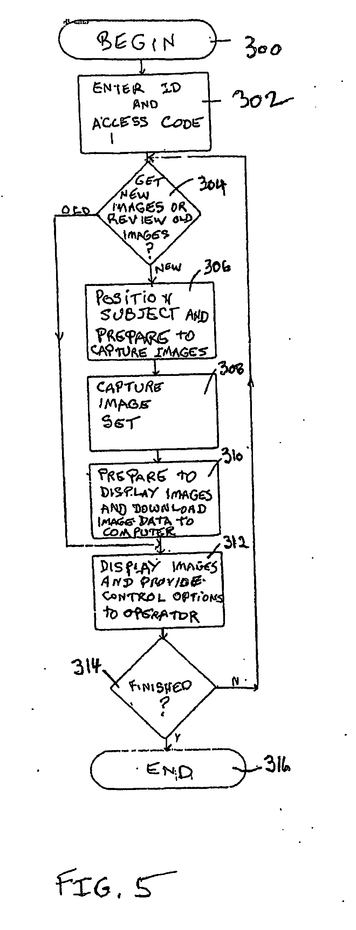

FIG. 5 shows exemplary processing steps that an exemplary embodiment of the present invention would utilize in obtaining and viewing the images of a subject S. The processing flow begins 300 by the operator entering an access code or an I.D. password 302. This is an optional step in the event that the imaging station 200 is utilized by more than one person. For example, if the imaging station were a public facility, the individuals who utilize it would prefer that their images would remain private and accessible only by them upon entry of a suitable password/access code. In the event that the imaging station 200 is a private facility, for example, located in the home of the user, then it would not be necessary for a password to be utilized.

At the beginning of the session, it is determined 304 whether the purpose of the session is to get new images or to review old images. This can be implemented by simply sensing on the control buttons 228b and 228c which provide the user with these options. If the user elects to take new images, for example, by touching control button 228c, the imaging station 200 provides instructions to the subject S to position themselves and to prepare to capture the images 306. For example, the subject may be instructed to approach the camera such that their face fills the field of view as shown in thumbnail 224a. Once positioned, the subject is instructed to turn off the fluorescent lights and/or to close their eyes to prevent the flashes from being seen. Once the subject S indicates a ready state, e.g., by pressing a button, e.g., 228c for taking pictures or by the system sensing that the subject S has turned off the fluorescent lights, the image set can be captured 308 by sequentially taking pictures utilizing different flashes and filters. The images are then prepared to be displayed and downloaded 310 to the computer of the imaging station 200. Once the images have been captured and downloaded onto the system computer, the images may be displayed 312 on the monitor 270 and the operator is provided with various control options. When the operator is finished 314 reviewing the images, the operation is at an end 316, otherwise, the user may obtain additional new images or review old images.

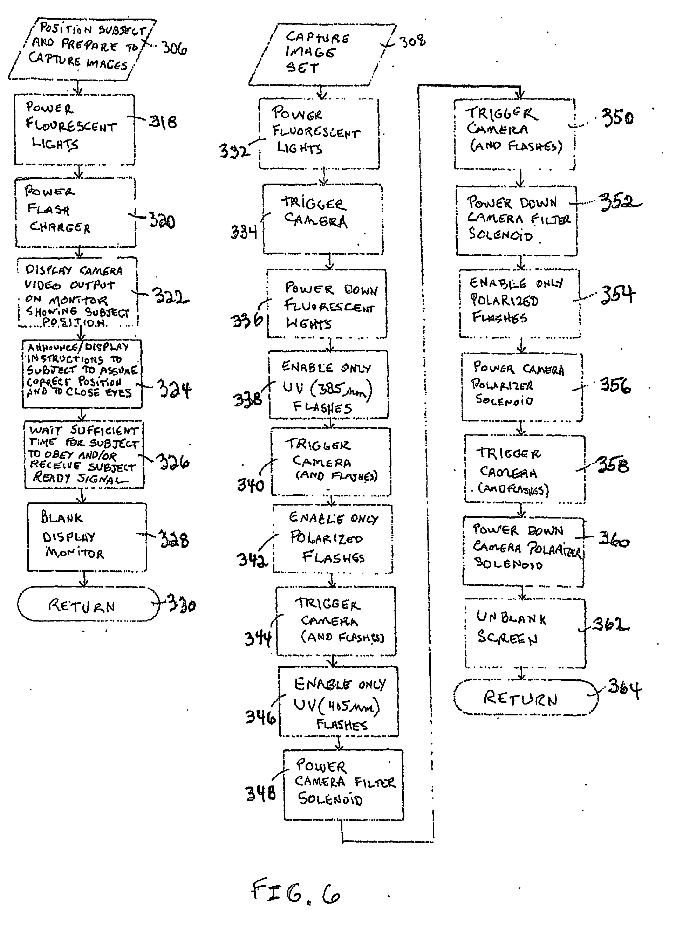

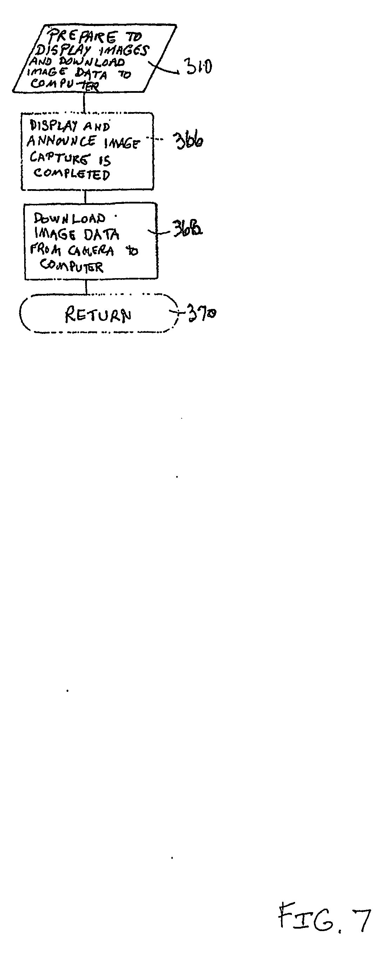

FIGS. 6 and 7 show the basic process shown in FIG. 5, in greater detail, namely, the step of positioning and preparing 306 to capture images includes powering 318 the fluorescent lights, powering 320 the flash charger and displaying 322 the camera video output on the monitor 270 so that the subject S may ascertain their position relative to the camera 322. As described above, this image is shown in thumbnail 224a or elsewhere as determined by the programmer. The program announces and/or displays 324 instructions to the user/subject to place themselves at the correct position relative to the camera and to close their eyes. A delay is provided 326, to give the subject S sufficient time to obey the commands for positioning and closing of the eyes and/or the computer program enables 326 a subject-ready signal, such as the subject pressing control button 228C to initiate taking pictures. The display monitor 270 is blanked 328 to avoid interfering with the illuminating light, and a return of control 330 to the main processing flow is executed. In capturing 308, the image set, the fluorescent lights 217a, 217b are powered 332 and the camera 211 is triggered 334. The fluorescent lights 217a, 217b are then powered down 336 and the 385 nanometer UV flashes 240a, 240b are enabled 338. The camera is then triggered 340 causing the 385 nanometer UV flashes 240a, 240b to flash. The polarized flashes 220a, 220b are enabled 342 and the camera 211 is triggered, triggering 344 the polarized flashes 220a, 22b. The 405 nanometer flashes 230a, 230b are enabled 346 and the camera filter solenoid 280b is powered 348, thereby moving the filter 281a in front of the camera 211. The camera 211 is then triggered 350 along with the flashes 230a, 230b. The filter 281a is then removed from a position in front of the camera 211 by powering down 352 the filter solenoid 280b. The polarized flashes 220a, 220b are enabled 354 and the camera polarizer solenoid 280a is powered 356 thereby moving the polarizing filter 281b in front of the camera 211. The camera 211 is then triggered, flashing 358 the polarized flashes 220a, 220b. The camera polarizer solenoid 280a is powered down 360 to remove the polarizing filter 281b away from the front of the camera 211. The display screen 270 is unblanked 362 and control returns 364 to the main processing flow.

To prepare to display images and download 310 the image data to the computer 552 (Fig. 9), the computer program announces 366 that the image capture is complete and then the image data is downloaded 368 from the camera 211 to the computer 552, where it is stored in memory or on a memory storage device. Control returns 370 to the main processing flow.

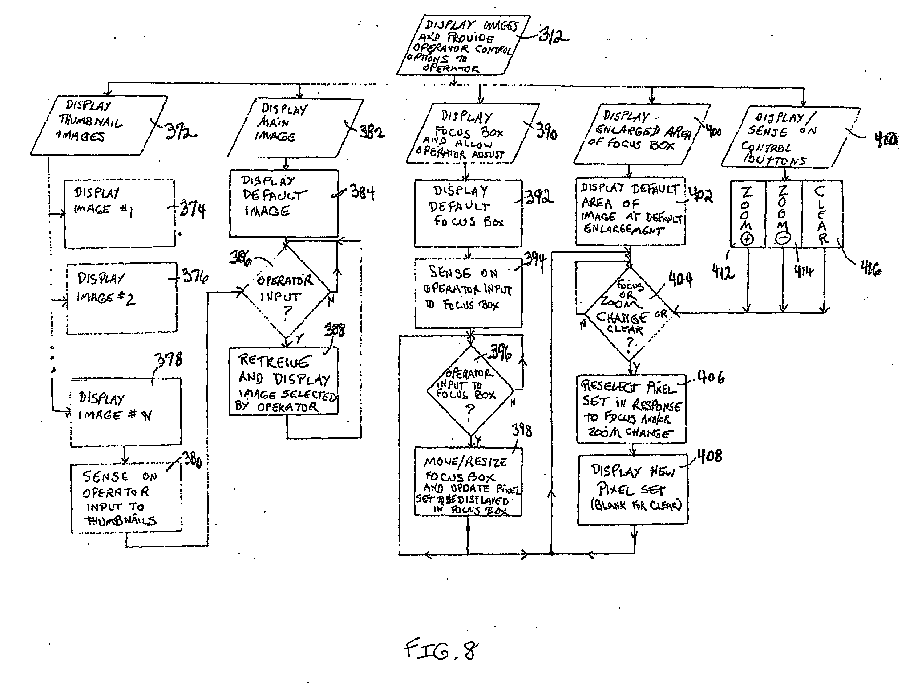

FIG. 8 shows exemplary processing associated with displaying 312 images and providing operator control options to the operator/subject S. As noted above, the display 270 has a plurality of thumbnail images 224a through 224e. Any number of thumbnail images may be displayed 372 as indicated by the dotted lines and reference to display 374 image #1, display 376 image #2 and display 378 image in. Because the display monitor 270 is preferably a touch screen monitor, the thumbnail display areas 224a through 224e can be used to sense on 380 operator input (touching the particular thumbnail image 224a-224b that the operator wishes to display in the main display area 225,). In displaying 382 the main image, a default image is displayed 384 which would typically be the first image taken, e.g., an image taken in white light or fluorescent light. Based upon the operator input 386 (touching a particular thumbnail image), the image associated with that particular thumbnail 224b through 224e is retrieved 388 from memory or from a storage device and the image is displayed in the main image area 225. As noted above, a focus box 226 is displayed 390 in the main image area 225. A default focus box 226 is displayed 392 first and then the program senses 394 upon operator input to the focus box 226 for changes. More particularly, if the operator/subject S touches the display screen 270 within the boundaries of the focus box 226 and retains contact with it while moving the contacting finger across the screen 270, the focus box 226 can be dragged to the area of the image of interest to the operator/subject S. Operator inputs to the focus box location are monitored at step 396. If there has been a change, then the pixel set associated with the focus box 226 is updated 398. The magnified image associated with the focus box 226 is displayed 400 and a default area associated with a default position of the focus box 226 is displayed 402 first. The program continually senses 404 upon changes in focus or zoom or for "clear" signals that are generated by the operator touching one of the control buttons 228d, 228e, 228f. Any of these inputs causes the pixel set associated with the focus box to be reselected 406 and displayed 408 in order to implement a focus or zoom change and/or for clearing the display. At Step 410, the control buttons "+", "-" and "clear" are displayed and sensed upon. As noted above, touching 412 "+" results in enlargement of the image displayed in the enlarged display area 227. Pressing 414 "-" results in a lower magnification and pressing 416 "clear" results in the enlarged display area 227 being blanked.

FIG. 9 shows an exploded view of an embodiment of the present invention in which, imaging station 500 has center housing 502 containing a plurality of power supplies 532, 533, 534 for powering the various system elements, including the monitor 570 and the system computer 552, as well as the camera 511. The camera 511 is mounted on an intermediate panel 543 which also serves to provide a structure for mounting the monitor 570. The intermediate panel 543 is received in and attached to the housing 502 by conventional fasteners, such as threaded fasteners or rivets. Front panel 542 is attached to the housing 502 and/or intermediate panel 543. The front panel 542 has apertures for camera 511, speaker 518 and monitor 570. Only one door assembly 504a is shown. Either single or plural door configurations are within the scope of the present invention, as are configurations where the door(s) open up or down.

FIG. 10 shows the imaging station 500 of FIG. 9 in a closed condition with door panels 504a and 504b closed, such that the monitor 570 is not visible. This embodiment of the imaging station 500 has some similarities to a conventional medicine cabinet in that it can be mounted on a wall and the doors 504a, 504b closed to protect the interior contents of the housing 502. The front surfaces of the door assemblies 504a, 504b may be mirrored.

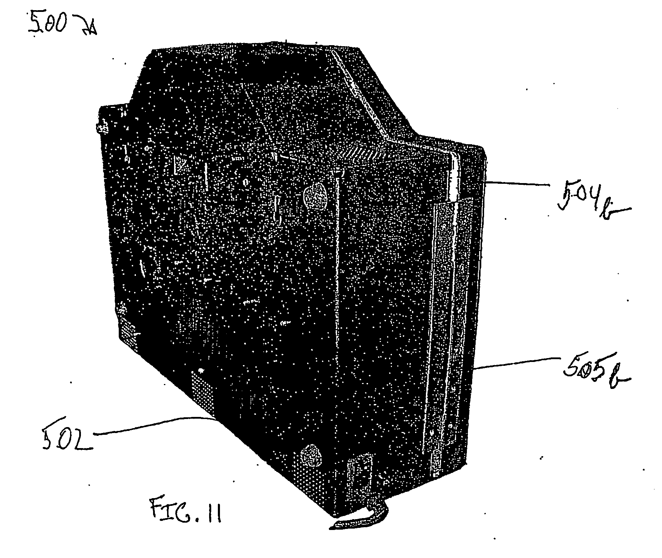

FIG. 11 is a rear view of the imaging station 500 illustrating that the door assemblies 504a, 504b are hingedly attached to the housing 502 by hinges e.g., 505b.

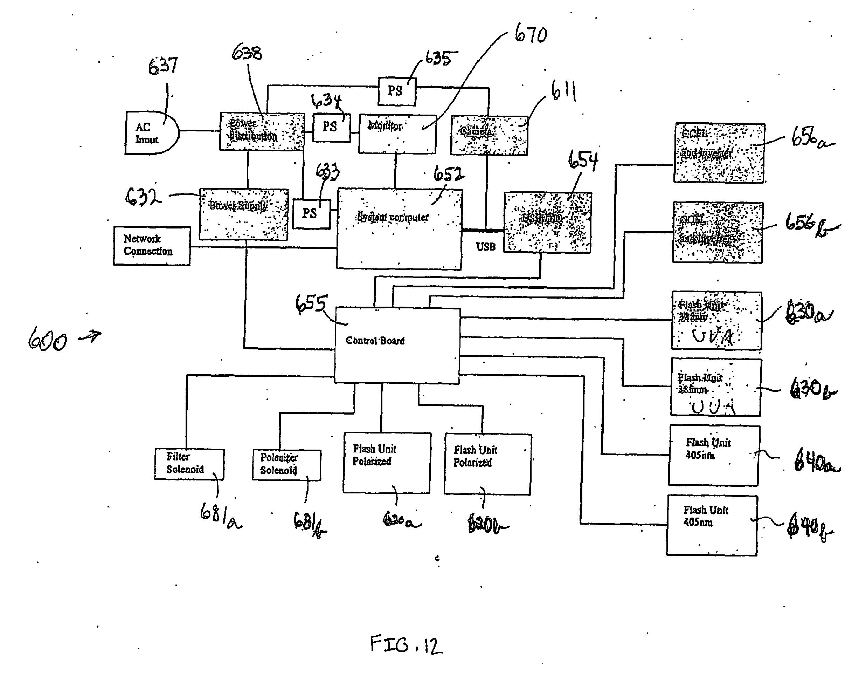

FIG. 12 is a block diagram illustrating the electrical components of the imaging station 600. As can be appreciated, AC input 637 is routed to a power distribution box 638 for spreading the AC input to a plurality of power supplies 632, 633, 634, 635, which power control board 655, computer 652, monitor 670 and camera 611, respectively. Camera 611 communicates with the computer 652 via a U.S.B. data input/output 654 and thereby to transfer images to the control board 655. The control board 655 controls filter solenoids 681a, 681b, flash unit polarizers 620a, 620b, CCFLs and inverters 656a, 656b and flash units 630a, 630b, 640a and 640b.

While the present invention has been explained above in terms of an apparatus and method employing multiple images captured under different lighting conditions e.g., different wavelengths of light, the present invention would still be applicable to the capture and analysis of even a single image taken under selected lighting conditions. For example, the imaging station 200 could be utilized to capture and analyze a single image taken with blue light.

It should be appreciated that the present invention 200 is intended to be operated by any person and therefore can be utilized in a private setting, such as the home, by the subject S.

It is understood that while the invention has been described in conjunction with the detailed description thereof, that the foregoing description is intended to illustrate and not limit the scope of the invention, which is defined by the scope of the appended claims. Other aspects, advantages, and modifications are within the scope of the claims.

## Claims

1. An apparatus for capturing images of a person, comprising:
a housing;
a camera mounted in said housing for capturing the images of the person;
means for illuminating mounted in said housing, said means for illuminating providing light for capturing the images of the person and being capable of providing at least two different wavelengths of light in capturing the images, the wavelengths of light used in capturing a first image differing from the wavelengths of light used in capturing a subsequent image;
a display mounted in said housing for displaying images, captured by said curnern;
control means for controlling said cameras, said means for illuminating and said display for selectively capturing and displaying the captured images on said display,
wherein said display is a touch-screen display capable of receiving input from an operator of the Apparatus four controlling the review of captured images in conjunction with said control means and has an image display area for displaying a captured image and a control area sensing on operator contact, **characterised in that**
the image display area is subdivided for displaying a plurality of different images permitting side-by-side comparison of the different images, which may each be enlarged and repositioned; the enlargement and repositioning of the images being synchronized such that cach image is presented in tho same position and scale as the others.

2. The apparatus of Claim 1, wherein said housing has a hollow portion receiving said camera and at least one door attached thereto for covering said camera when in a closed position and uncovering said camera when in an open position.

3. The apparatus of Claim 2, wherein said hollow receives said display and said at least one door covers said display when in the closed position.

4. The apparatus of Claim 3, further including a pair of doors flanking a central portion of said housing, said pair of doors hingedly connected to said central portion.

5. The apparatus of Claim 4, further including a reflective surface on an outside surface of one of said doors, said reflective surface functioning as a mirror.

6. The apparatus of Claim 5, wherein said reflective surface is disposed on the outside surface of said door when the door is in the closed position.

7. The apparatus of Claim 1, wherein said means for illuminating includes a plurality of different light sources.

8. The apparatus of Claim 7, wherein said means for illuminating includes a light filter for filtering light from at least one of said plurality of light sources.

9. the apparatus of Claim 8, wherein said light filter is positioned in front of the camera to filter light entering the camera.

10. The apparatus of Claim 9, wherein said filter is moveable between a filtering position in front of the camera and a non-filtering position away from the front of the camera.

11. The apparatus of Claim 8, wherein said filter is positioned in front or at least one of said plurality of light sources to filter light emanating therefrom.

12. The apparatus of Claim 8. wherein said light source includes a plurality of light fillers which affect wavelengths of light.

13. The apparatus of Claim 7, wherein said means for illuminating provides light selected from at least one of visible light, polarized light, ultraviolet light and blue light.

14. The apparatus of Claim 1, wherein said control area includes at least one thumbnail image corresponding to an image previously captured by said camera.

15. The apparatus of Claim 14, wherein said control area includes a plurality off thumbnail images corresponding to images previously captured by said camera, operator contact with which invokes said control means to display the corresponding image in the image display area.

16. The apparatus of Claim 1, wherein said touch-screen has an enlarged image area for displaying a portion of the image displayed in the image display area at a different magnification than in said image display area.

17. The apparatus of Claim 16, wherein the portion of the image displayed in the enlarged image area and the degree of enlargement is selectable by the operator.

18. The apparatus of Claim 1, wherein said touch-screen displays a real time, refreshed image captured hy said camera.

19. The apparatus of Claim 1, wherein said control area includes al least one of a button, a dial and a lever.

20. The apparatus of Claim 1, wherein the operator is the person whose image is captured by the apparatus.

21. The apparatus of Claim 1, wherein said control means includes a computer programmed to direct said camera and said means for illuminating to automatically sequentially capture a plurality of images using a plurality of different wavelengths of light.

22. The apparatus of Claim 21, wherein said light source includes a light attached to an inner portion of said at least one door such that when said at least one door is open, said light is capably off illuminating the person, the incidence angle of the illuminating light being adjustable by adjusting the angle of the at least one door relative to said hollow portion.

23. The Apparatus of Claim 22, further comprising means for positioning said at least one door at a selected position.

24. The apparatus of Claim 22, further including a polarizing filter juxtaposed next to said light attached to said door for providing polarized light for illumination of the subject.

25. The apparatus of Claim 1, further including means for providing usage instructions to a user.

26. The Apparatus of Claim 25, wherein the instructions provided by said means for providing usage instructions enable a person having no training in the use of the apparatus to use it to capture and view images taken thereby.

27. The apparatus of Claim 25, wherein said means for providing usage instructions includes u speaker mounted in said housing for audibly announcing usage instructions.

28. The apparatus of Claim 25, wherein said means for providing usage instructions includes said display which displays usage instructions.

29. The apparatus of Claim 1, wherein said display is directed at the person when the person is positioned for image capture.

30. The apparatus of Claim 1, wherein said light source includes at least one flash.

31. A method of capturing images of a person, comprising the steps of
(A) providing an imaging station having a housing, a camera mounted in said housing, a light source mounted in the housing, a displays mounted in the housing for displaying images captured by the camera, wherein said display is a touch-screen display has an image display area for displaying a captured images and a control area sensing on operator contact, wherein the image display area is subdivided; and a computer;
(B) positioning the person relative to the camera;
(C) automatically capturing a plurality of images with the camera;
(D) storing the images captured and;
(E) selectively displaying the captured images on the display
(F) receiving input from an operator of the apparatus for controlling the review of captured images in conjunction with said control means
(G) displaying a plurality of different images on the subdivided image display area permitting side-by-side comparaison of the different images, which may each he enlarged and repositioned, the enlargement and repositioning the images being synchronized such that each image is presented in the same position and scale as the others.

32. The method of Claim 31, wherein the imagine station has a plurality of sources of light of different frequencies and the plurality of different images includes images captured with different frequencies of light.

33. The method of Claim 32, further comprising the step of triggering the light sources sequentially as the different images are captured.

34. The method of Claim 33, wherein the at least one image is captured during the triggering of two different light sources.

35. The method of Claim 31, further comprising the step of providing instructions for self-use of the imaging station to the person whose image is captured by the imaging station.

36. The method of Claim 35, wherein the instructions are in the form of at least one of text and graphics.

37. The method of Claim 35, wherein the instructions given address interpreting the images captured.

38. Thu method of Claim 31, wherein the imaging station has at least one door covering the camera and further comprising the steps of carrying the imaging station to a selected location, placing the imagine station on a support surface and opening the at least one door prior to said step of positioning.

39. The method of Claim 37, wherein the at least one door has a light source in it and further comprising the step of adjusting the angle of the at least one door to adjust the angle of incidence of the light emanated by the light source on the person.

40. The method of Claim 31, wherein said step of positioning includes positioning any selected portion of the body before the camera.

41. The method or Claim 31 wherein said step of positioning includes capturing n real-time image or the person, displaying the real-time image on the display and allowing the Person to interactively adjust their position based upon the real-time image displayed.

42. The method of Claim 31, wherein the imaging station is operable by the person whose image is captured.

43. The method of Claim 31, wherein more than one of said plurality of images are displayed as thumbnail images on said display allowing the person to select one of said thumbnail Images for display in a larger size.

44. The method of Claim 31, further including the stops of selectively repositioning the image displayed on the display in large format and selectively zooming in and out on the image.

45. The method of Claim 31, wherein said steps of positioning and storming are conducted by the person whose image is captured and said steps of capturing and storing are conducted by the imaging station.

## Patentansprüche

1. Gerät zum Erfassen von Bildern einer Person, das Folgendes umfasst:
ein Gehäuse;
eine in dem Gehäuse montierte Kamera zum Erfassen der Bilder der Person;
in dem Gehäuse montierte Mittel zum Beleuchten, wobei die Mittel zum Beleuchten Licht zum Erfassen der Bilder der Person liefern und mindestens zwei verschiedene Lichtwellenlängen beim Erfassen der Bilder liefern können, wobei die beim Erfassen eines ersten Bilds verwendeten Lichtwellenlängen von dem beim Erfassen eines nachfolgenden Bilds verwendeten Lichtwellenlängen verschieden sind;
ein in dem Gehäuse montiertes Display zum Anzeigen von von der Kamera erfassten Bildern;
Steuermittel zum Steuern der Kamera, die Mittel zum Beleuchten und das Display zum selektiven Erfassen und Anzeigen der erfassten Bilder auf dem Display,
wobei das Display ein Touchscreen-Display ist, das eine Eingabe von einem Operator des Geräts empfangen kann zum Steuern der Überprüfung von erfassten Bildern in Verbindung mit den Steuermitteln und einen Bilddisplaybereich aufweist zum Anzeigen eines erfassten Bilds und einen bei Operatorkontakt erfassenden Steuerbereich, **dadurch gekennzeichnet, dass**
der Bilddisplaybereich unterteilt ist zum Anzeigen von mehreren verschiedenen Bildern, was einen Vergleich der verschiedenen Bilder Seite an Seite gestattet, die jeweils vergrößert und neu positioniert werden können;
wobei das Vergrößern und das Neupositionieren der Bilder derart synchronisiert wird, dass jedes Bild in der gleichen Position und im gleichen Maßstab wie die anderen präsentiert wird.

2. Gerät nach Anspruch 1, wobei das Gehäuse einen hohlen Abschnitt aufweist, der die Kamera aufnimmt, und mindestens eine daran angebrachte Tür zum Bedecken der Kamera in einer geschlossenen Position und Aufdecken der Kamera in einer offenen Position.

3. Gerät nach Anspruch 2, wobei die Aushöhlung das Display aufnimmt und die mindestens eine Tür in der geschlossenen Position das Display bedeckt.

4. Gerät nach Anspruch 3, weiterhin mit einem Paar von Türen, die einen zentralen Abschnitt des Gehäuses flankieren, wobei das Paar von Türen an den zentralen Abschnitt angelenkt ist.

5. Gerät nach Anspruch 4, weiterhin mit einer reflektierenden Oberfläche auf einer äußeren Oberfläche einer der Türen, wobei die reflektierende Oberfläche als ein Spiegel fungiert.

6. Gerät nach Anspruch 5, wobei die reflektierende Oberfläche auf der äußeren Oberfläche der Tür angeordnet ist, wenn sich die Tür in der geschlossenen Position befindet.

7. Gerät nach Anspruch 1, wobei das Mittel zum Beleuchten mehrere verschiedene Lichtquellen enthält.

8. Gerät nach Anspruch 7, wobei das Mittel zum Beleuchten einen Lichtfilter zum Filtern von Licht von mindestens einer der mehreren Lichtquellen enthält.

9. Gerät nach Anspruch 8, wobei der Lichtfilter vor der Kamera positioniert ist, um in die Kamera eintretendes Licht zu filtern.

10. Gerät nach Anspruch 9, wobei der Filter zwischen einer Filterungsposition vor der Kamera und einer Nicht-Filterungsposition weg von der Vorderseite der Kamera bewegt werden kann.

11. Gerät nach Anspruch 8, wobei der Filter vor mindestens einer der mehreren Lichtquellen positioniert ist, um daraus austretendes Licht zu filtern.

12. Gerät nach Anspruch 8, wobei die Lichtquelle mehrere Lichtfilter enthält, die Lichtwellenlängen beeinflussen.

13. Gerät nach Anspruch 7, wobei das Mittel zum Beleuchten Licht bereitstellt, ausgewählt unter sichtbarem Licht, polarisiertem Licht, Ultraviolettlicht und/oder blauem Licht.

14. Gerät nach Anspruch 1, wobei der Steuerbereich mindestens ein Miniaturbild enthält, das einem zuvor von der Kamera erfassten Bild entspricht.

15. Gerät nach Anspruch 14, wobei der Steuerbereich mehrere Miniaturbilder enthält, die zuvor von der Kamera erfassten Bildern entsprechen, wobei ein Operatorkontakt damit das Steuermittel aufruft, das entsprechende Bild in dem Bilddisplaybereich anzuzeigen.

16. Gerät nach Anspruch 1, wobei der Touchscreen einen vergrößerten Bildbereich aufweist zum Anzeigen eines Teils des in dem Bildanzeigebereich angezeigten Bilds mit einer anderen Vergrößerung als in dem Bildanzeigebereich.

17. Gerät nach Anspruch 16, wobei der in dem vergrößerten Bildbereich angezeigte Teil des Bilds und der Vergrößerungsgrad von dem Operator gewählt werden können.

18. Vorrichtung nach Anspruch 1, wobei der Touchscreen ein von der Kamera erfasstes aktualisiertes Echtzeitbild anzeigt.

19. Gerät nach Anspruch 1, wobei der Steuerbereich einen Knopf, eine Rundskala und/oder einen Hebel enthält.

20. Gerät nach Anspruch 1, wobei der Operator die Person ist, deren Bild von dem Gerät erfasst wird.

21. Gerät nach Anspruch 1, wobei das Steuermittel einen Computer enthält, der programmiert ist, die Kamera und das Mittel zum Beleuchten zu lenken, unter Verwendung von mehreren verschiedenen Lichtwellenlängen mehrere Bilder automatisch und sequenziell zu erfassen.

22. Gerät nach Anspruch 21, wobei die Lichtquelle ein an einem inneren Abschnitt der mindestens einen Tür derart angebrachtes Licht enthält, so dass, wenn die mindestens eine Tür offen ist, das Licht die Person beleuchten kann, wobei der Einfallswinkel des beleuchtenden Lichts durch Verstellen des Winkels der mindestens einen Tür relativ zu dem hohlen Abschnitt verstellt werden kann.

23. Gerät nach Anspruch 22, weiterhin umfassend Mittel zum Positionieren der mindestens einen Tür in einer ausgewählten Position.

24. Gerät nach Anspruch 22, weiterhin mit einem Polarisierungsfilter, der benachbart neben dem Licht ist, der an der Tür angebracht ist, zum Bereitstellen von polarisiertem Licht zur Beleuchtung des Objekts.

25. Gerät nach Anspruch 1, weiterhin mit einem Mittel zum Liefern von Benutzungsanweisungen an einen Benutzer.

26. Gerät nach Anspruch 25, wobei die von dem Mittel zum Liefern von Benutzungsanweisungen gelieferten Anweisungen es einer Person ohne Training bei der Verwendung des Geräts ermöglichen, es zum Erfassen von Bildern und zum Betrachten von damit erfassten Bildern zu verwenden.

27. Gerät nach Anspruch 25, wobei das Mittel zum Liefern von Benutzungsanweisungen einen in dem Gehäuse montierten Lautsprecher zum hörbaren Ankündigen von Benutzungsanweisungen enthält.

28. Gerät nach Anspruch 25, wobei das Mittel zum Liefern von Benutzungsanweisungen das Display enthält, das Benutzungsanweisungen anzeigt.

29. Gerät nach Anspruch 1, wobei das Display auf die Person gerichtet ist, wenn die Person zur Bilderfassung positioniert ist.

30. Gerät nach Anspruch 1, wobei die Lichtquelle mindestens einen Blitz enthält.

31. Verfahren zum Erfassen von Bildern einer Person, das die folgenden Schritte umfasst:
(A) Bereitstellen einer Bildgebungsstation mit einem Gehäuse, einer in dem Gehäuse montierten Kamera, einer in dem Gehäuse montierten Lichtquelle, einem in dem Gehäuse montierten Display zum Anzeigen von von der Kamera erfassten Bildern, wobei das Display ein Touchscreen-Display ist und einen Bilddisplaybereich zum Anzeigen eines erfassten Bilds und einen bei Operatorkontakt erfassenden Steuerbereich aufweist, wobei der Bilddisplaybereich unterteilt ist; und einem Computer;
(B) Positionieren der Person relativ zu der Kamera;
(C) automatisches Erfassen von mehreren Bildern mit der Kamera;
(D) Speichern der erfassten Bilder und
(E) selektives Anzeigen der erfassten Bilder auf dem Display;
(F) Empfangen einer Eingabe von einem Operator des Geräts zum Steuern der Überprüfung von erfassten Bildern in Verbindung mit dem Steuermittel;
(G) Anzeigen mehrerer verschiedener Bilder auf dem unterteilten Bilddisplaybereich, was einen Vergleich der verschiedenen Bilder Seite an Seite gestattet, die jeweils vergrößert und neu positioniert werden können, wobei das Vergrößern und Neupositionieren der Bilder derart synchronisiert ist, dass jedes Bild in der gleichen Position und mit dem gleichen Maßstab wie die anderen präsentiert wird.

32. Verfahren nach Anspruch 31, wobei die Bildgebungsstation mehrere Lichtquellen mit unterschiedlichen Frequenzen aufweist und die mehreren verschiedenen Bilder mit verschiedenen Lichtfrequenzen erfasste Bilder beinhalten.

33. Verfahren nach Anspruch 32, weiterhin mit dem Schritt des sequenziellen Auslösens der Lichtquellen, wenn die verschiedenen Bilder erfasst werden.

34. Verfahren nach Anspruch 33, wobei das mindestens eine Bild während des Auslösens von zwei verschiedenen Lichtquellen erfasst wird.

35. Verfahren nach Anspruch 31, weiterhin mit dem Schritt des Lieferns von Anweisungen zur Eigenverwendung der Bildgebungsstation an die Person, deren Bild von der Bildgebungsstation erfasst wird.

36. Verfahren nach Anspruch 35, wobei die Anweisungen in Form von Text und/oder Grafik vorliegen.

37. Verfahren nach Anspruch 35, wobei die angegebenen Anweisungen ein Interpretieren der erfassten Bilder betreffen.

38. Verfahren nach Anspruch 31, wobei die Bildgebungsstation mindestens eine die Kamera bedeckende Tür aufweist, und weiterhin umfassend die Schritte des Tragens der Bildgebungsstation zu einem gewählten Ort, Platzieren der Bildgebungsstation auf einer Stützoberfläche und Öffnen der mindestens einen Tür vor dem Schritt des Positionierens.

39. Verfahren nach Anspruch 37, wobei die mindestens eine Tür eine Lichtquelle in sich aufweist, und weiterhin umfassend den Schritt des Verstellens des Winkels der mindestens einen Tür, um den Einfallswinkel des von der Lichtquelle auf die Person austretenden Lichts zu verstellen.

40. Verfahren nach Anspruch 31, wobei der Schritt des Positionierens das Positionieren eines beliebigen gewählten Teils des Körpers vor der Kamera beinhaltet.

41. Verfahren nach Anspruch 31, wobei der Schritt des Positionierens das Erfassen eines Echtzeitbilds der Person, das Anzeigen des Echtzeitbilds auf dem Display und das Gestatten, dass die Person ihre Position auf der Basis des angezeigten Echtzeitbilds interaktiv verstellt, beinhaltet.

42. Verfahren nach Anspruch 31, wobei die Bildgebungsstation von der Person betätigt werden kann, deren Bild erfasst wird.

43. Verfahren nach Anspruch 31, wobei mehr als eines der mehreren Bilder als Miniaturbilder auf dem Display angezeigt werden, wodurch die Person eines der Miniaturbilder zur Anzeige in einer größeren Größe wählen kann.

44. Verfahren nach Anspruch 31, weiterhin mit dem Schritt des selektiven Neupositionierens des auf dem Display angezeigten Bilds in einem größeren Format und dem Schritt des selektiven Hereinzoomens und Herauszoomens aus dem Bild.

45. Verfahren nach Anspruch 31, wobei der Schritt des Positionierens und der Schritt des Speicherns von der Person durchgeführt werden, deren Bild erfasst wird, und der Schritt des Erfassens und der Schritt des Speicherns von der Bildgebungsstation durchgeführt werden.

## Revendications

1. Appareil de saisie d'images d'une personne, comprenant :
un boîtier ;
un appareil photographique monté dans ledit boîtier pour saisir les images de la personne ;
un moyen d'éclairage monté dans ledit boîtier, ledit moyen d'éclairage produisant une lumière pour saisir les images de la personne et pouvant produire au moins deux longueurs d'onde de lumière différentes lors de la saisie des images, les longueurs d'onde de lumière utilisées dans la saisie d'une première image différant des longueurs d'onde de lumière utilisées dans la saisie d'une image suivante ;
un affichage monté dans ledit boîtier pour afficher les images saisies par ledit appareil photographique ;
un moyen de commande pour commander ledit appareil photographique, ledit moyen d'éclairage et ledit affichage afin de saisir et d'afficher sélectivement les images saisies sur ledit affichage,
dans lequel ledit affichage est un affichage à écran tactile capable de recevoir une entrée depuis un opérateur de l'appareil pour commander le passage en revue des images saisies conjointement avec ledit moyen de commande et comporte une zone d'affichage d'image pour afficher une image saisie et une zone de commande détectant un contact par l'opérateur,
dans lequel la zone d'affichage d'image est subdivisée pour afficher une pluralité d'images différentes permettant la comparaison côte à côte des images différentes, lesquelles peuvent être chacune agrandies et repositionnées ;
**caractérisé en ce que** l'appareil est adapté de telle sorte que l'agrandissement et le repositionnement des images soient synchronisés afin que chaque image soit présentée à la même position et à la même échelle que les autres.

2. Appareil selon la revendication 1, dans lequel ledit boîtier comporte une partie creuse recevant ledit appareil photographique et au moins un volet fixé à celui-ci pour, en position fermée, recouvrir ledit appareil photographique et, en position ouverte, découvrir ledit appareil photographique.

3. Appareil selon la revendication 2, dans lequel ledit creux reçoit ledit affichage et ledit au moins un volet en position fermée recouvre ledit affichage.

4. Appareil selon la revendication 3, comportant en outre une paire de volets flanquant une partie centrale dudit boîtier, ladite paire de volets étant fixée de façon articulée à ladite partie centrale.

5. Appareil selon la revendication 4, comportant en outre une surface réfléchissante sur une surface extérieure de l'un desdits volets, ladite surface réfléchissante servant de miroir.

6. Appareil selon la revendication 5, dans lequel ladite surface réfléchissante est disposée sur la surface extérieure dudit volet quand le volet est en position fermée.

7. Appareil selon la revendication 1, dans lequel ledit moyen d'éclairage comporte une pluralité de sources de lumière différentes.

8. Appareil selon la revendication 7, dans lequel ledit moyen d'éclairage comporte un filtre de lumière pour filtrer la lumière provenant d'au moins l'une de ladite pluralité de sources de lumière.

9. Appareil selon la revendication 8, dans lequel ledit filtre de lumière est positionné devant la caméra afin de filtrer la lumière entrant dans l'appareil photographique.

10. Appareil selon la revendication 9, dans lequel ledit filtre est déplaçable entre une position filtrante devant l'appareil photographique et une position non filtrante éloignée du devant de l'appareil photographique.

11. Appareil selon la revendication 8, dans lequel ledit filtre est positionné devant au moins l'une de ladite pluralité de sources de lumière afin de filtrer la lumière en provenant.

12. Appareil selon la revendication 8, dans lequel ladite source de lumière comporte une pluralité de filtres de lumière qui affectent les longueurs d'onde de lumière.

13. Appareil selon la revendication 7, dans lequel ledit moyen d'éclairage produit une lumière sélectionnée parmi la lumière visible, la lumière polarisée, la lumière ultraviolette et la lumière bleue.

14. Appareil selon la revendication 1, dans lequel ladite zone de commande comporte au moins une imagette correspondant à une image saisie précédemment par ledit appareil photographique.

15. Appareil selon la revendication 14, dans lequel ladite zone de commande comporte une pluralité d'imagettes correspondant à des images saisies précédemment par l'appareil photographique, un contact par l'opérateur sur celles-ci amenant ledit moyen de commande à afficher l'image correspondante dans la zone d'affichage d'image.

16. Appareil selon la revendication 1, dans lequel ledit écran tactile comporte une zone d'image agrandie pour afficher une partie de l'image affichée dans la zone d'affichage d'image à une grandeur différente de celle dans ladite zone d'affichage d'image.

17. Appareil selon la revendication 16, dans lequel la partie de l'image affichée dans la zone d'image agrandie et le degré d'agrandissement sont sélectionnables par l'opérateur.

18. Appareil selon la revendication 1, dans lequel ledit écran tactile affiche une image rafraîchie en temps réel saisie par ledit appareil photographique.

19. Appareil selon la revendication 1, dans lequel ladite zone de commande comporte au moins l'un d'un bouton, d'un composeur et d'un levier.

20. Appareil selon la revendication 1, dans lequel l'opérateur est la personne dont l'image est saisie par l'appareil photographique.

21. Appareil selon la revendication 1, dans lequel ledit moyen de commande comporte un ordinateur programmé pour instruire ledit appareil photographique et ledit moyen d'éclairage afin de saisir automatiquement séquentiellement une pluralité d'images en utilisant une pluralité de longueurs d'onde de lumière différentes.

22. Appareil selon la revendication 21, dans lequel ladite source de lumière comporte une lumière fixée à une partie interne dudit au moins un volet de telle sorte que lorsque ledit au moins un volet s'ouvre, ladite lumière puisse éclairer la personne, l'angle d'incidence de la lumière d'éclairage étant réglable en réglant l'angle de l'au moins un volet par rapport à ladite partie creuse.

23. Appareil selon la revendication 22, comprenant en outre un moyen de positionnement dudit au moins un volet à une position sélectionnée.

24. Appareil selon la revendication 22, comportant en outre un filtre de polarisation juxtaposé à ladite lumière fixé audit volet pour produire une lumière polarisée pour l'éclairage du sujet.

25. Appareil selon la revendication 1, comportant en outre un moyen de délivrance d'instructions d'emploi à un utilisateur.

26. Appareil selon la revendication 25, dans lequel les instructions délivrées par ledit moyen de délivrance d'instructions d'emploi permettent à une personne sans formation dans l'usage de l'appareil de l'utiliser afin de saisir et de visionner les images prises par celui-ci.

27. Appareil selon la revendication 25, dans lequel ledit moyen de délivrance d'instructions d'emploi comporte un haut-parleur monté dans ledit boîtier pour annoncer de façon audible les instructions d'emploi.

28. Appareil selon la revendication 25, dans lequel ledit moyen de délivrance d'instructions d'emploi comporte ledit affichage qui affiche les instructions d'emploi.

29. Appareil selon la revendication 1, dans lequel ledit affichage est dirigé vers la personne quand la personne est positionnée pour la saisie d'images.

30. Appareil selon la revendication 1, dans lequel ladite source de lumière comporte au moins un flash.

31. Procédé de saisie d'images d'une personne, comprenant les étapes suivantes :
(A) fourniture d'une station de visionnement comportant un boîtier, un appareil photographique monté dans ledit boîtier, une source de lumière montée dans le boîtier, un affichage monté dans le boîtier pour afficher des images saisies par l'appareil photographique, ledit affichage étant un affichage à écran tactile comportant une zone d'affichage d'image pour afficher une image saisie et une zone de commande détectant un contact par l'opérateur, l'affichage d'image étant subdivisé, et un ordinateur ;
(B) le positionnement de la personne par rapport à l'appareil photographique ;
(C) la saisie automatique d'une pluralité d'images avec l'appareil photographique ;
(D) la mémorisation des images saisies ; et
(E) l'affichage sélectif sur l'affichage des images saisies
(F) la réception d'une entrée depuis un opérateur de l'appareil pour commander le passage en revue des images saisies conjointement avec ledit moyen de commande
(G) l'affichage d'une pluralité d'images différentes sur la zone d'affichage d'image subdivisée permettant la comparaison côte à côte des images différentes, lesquelles peuvent être chacune agrandies et repositionnées, l'agrandissement et le repositionnement des images étant synchronisés afin que chaque image soit présentée à la même position et à la même échelle que les autres.

32. Procédé selon la revendication 31, dans lequel la station de visionnement comporte une pluralité de sources de lumière de différentes fréquences et la pluralité d'images différentes comporte des images saisies avec différentes fréquences de lumière.

33. Procédé selon la revendication 32, comprenant en outre l'étape de déclenchement séquentiel des sources de lumière au fur et à mesure de la saisie des images différentes.

34. Procédé selon la revendication 33, dans lequel l'au moins une image est saisie au déclenchement de deux sources de lumière différentes.

35. Procédé selon la revendication 31, comprenant en outre l'étape de délivrance d'instructions d'emploi de la station de visionnement à la personne dont l'image est saisie par la station de visionnement.

36. Procédé selon la revendication 35, dans lequel les instructions se présentent sous forme au moins de texte et de graphiques.

37. Procédé selon la revendication 35, dans lequel les instructions données permettent l'interprétation des images saisies.

38. Procédé selon la revendication 31, dans lequel la station de visionnement comporte au moins un volet recouvrant la caméra et comprenant en outre les étapes de transport de la station de visionnement à un emplacement sélectionné, le placement de la station de visionnement sur une surface de support et l'ouverture dudit au moins un volet avant ladite étape de positionnement.

39. Procédé selon la revendication 37, dans lequel ledit au moins un volet comporte à l'intérieur une source de lumière et comprenant en outre l'étape de réglage de l'angle de l'au moins un volet afin de régler l'angle d'incidence de la lumière émise par la source de lumière sur la personne.

40. Procédé selon la revendication 31, dans lequel ladite étape de positionnement comporte le positionnement de toute partie sélectionnée du corps devant l'appareil photographique.

41. Procédé selon la revendication 31, dans lequel ladite étape de positionnement comporte la saisie d'une image en temps réel de la personne, l'affichage de l'image en temps réel sur l'affichage et la possibilité pour la personne de régler interactivement sa position d'après l'image en temps réel affichée.

42. Procédé selon la revendication 31, dans lequel la station de visionnement est actionnable par la personne dont l'image est saisie.

43. Procédé selon la revendication 31, dans lequel plus d'une de ladite pluralité d'images sont affichées sous forme d'imagettes sur ledit affichage permettant à la personne de sélectionner l'une desdites imagettes en vue de l'afficher dans un format agrandi.

44. Procédé selon la revendication 31, comportant en outre les étapes de repositionnement sélectif de l'image affichée sur l'affichage dans un grand format et le grossissement ou la réduction sélectif de l'image.

45. Procédé selon la revendication 31, dans lequel lesdites étapes de positionnement et de mémorisation sont effectuées par la personne dont l'image est saisie et lesdites étapes de saisie et de mémorisation sont effectuées par la station de visionnement.
